# EUROPEAN PATENT SPECIFICATION

(11) **EP 1 330 212 B1**
(45) Date of publication and mention of the grant of the patent: **31.08.2005**
(21) Application number: 01961429.6
(22) Date of filing: 21.06.2001
(51) Int. Cl.: A61F 2/14

(54) **ORBITAL IMPLANT**
ORBITALIMPLANTAT
IMPLANT ORBITAL

(30) Priority: 29.09.2000 US 236343 P
(43) Date of publication of application: 30.07.2003
(73) Proprietor: Grip, Maine, 0765 Oslo (NO)
(72) Inventor: Grip, Maine, 0765 Oslo (NO)
(74) Representative: Briddes, Sam
(86) International application number: PCT/NO2001/000262
(87) International publication number: WO 2002/026166

(56) References cited:
- US-A- 2 661 480
- US-A- 4 731 077
- US-A- 5 330 529
- US-A- 5 466 258

## Description

This invention relates to an orbital implant and method, and more particularly to a supplemental orbital implant shaped to reduce the soft tissue deformity of the eye socket attributed to enophthalmos.

### Background art

When an eye is removed due to trauma, tumor or other disease states, the eyeball is usually replaced with a spherical implant made of either silicone or hydroxyapatite, such as that from United States patent 4,976,731. After surgical healing, an artificial eye prosthesis is prepared in either glass or plastic material. In the case of hydroxyapatite implants, the eye muscles can be sutured directly to the implant and thus confer movement to the implant. By a special peg attachment, this movement can be transferred to the artificial eye prosthesis and allows it to move in conjunction with the companion eye. A natural, lifelike movement of the artificial eye is thus possible and offers many benefits to patients wearing an artificial eye. Also, the peg supports the weight of the artificial eye and reduces pressure and strain on the lower eyelid.

Despite maximal efforts to create a well adapted and well functioning artificial eye connected to a tissue-integrated hydroxyapatite spherical implant, the majority of patients over time take on an unnatural shrunken appearance of the eyelid and orbit. This condition is due to atrophy or sinking back of the soft tissues of the orbit and is referred to as enophthalmos. Clinically, enophthalmos is specially manifested by a finding of deep superior sulcus in the form of a hollowing out of the upper eyelid and retarded position of the artificial eye prosthesis. Increasing the size of the ocular prosthesis such as shown in United States patent 5,466,258 can to a certain extent mitigate this disfiguring. However, the increased weight of a larger prosthesis adds to the strain on the lower eyelid and is seldom cosmetically successful.

### Summary of the invention

The present invention as described in claim 1 is intended to overcome the above-described problems. The implant according to the invention is designed to be a supplement to orbital implants currently in use, by compensating for the loss of soft tissue bulk in the eye socket. The implant is made of either polyethylene, polymethylmetacrylate, Teflon@ or other suitable material. The implant comprises an approximately 1.5 cm x 3-4 cm plate, approximately 2mm thick, adapted to the shape of the orbital floor. The plate has a plurality of attachment holes and a posterior, generally spherical projection, said projection providing added volume replacement for the lost soft tissue volume resulting from eye enucleation. The projection further exerts a forward pressure on the existing soft tissue, spherical ocular implant, eye prosthesis and eyelids, thus helping to eliminate the unnatural sunken appearance. The implant may be produced in a plurality of standard sizes adapted to varying degrees of volume replacement, and may be shaped by the surgeon according to the situation. In an alternate aspect of the invention, the implant could be utilized where the patient's natural eye, for one reason or another, lies deeper in the eye socket than normal.

The implant according to the invention may be utilized as part of an initial procedure, or as a corrective measure for an earlier, now unsatisfactory procedure. The method according to the invention comprises determining and selecting the appropriate standard-sized implant, modifying the size or shape of the projection as necessary, adjusting the length of the implant by cutting the anterior part of the plate, and the subperiosteal (via a subciliary incision in the lower eyelid) implantation of the implant in the floor of the orbit. The implant is securely fixed to the orbital floor by two stainless-steel ligatures that are anchored to drill holes in the inferior orbital rim, or by screws. Due to the bony fixation and the enwrapment in a subperiosteal pocket, the implant is totally immobilized and is not the subject of post-operative drifting or dislocation. The implant according to the invention is ideally combined with a spherical hydroxyapatite ocular implant, which during the last decade has gained increased popularity among ophthalmologists around the world. Together, these two implants offer significant esthetic improvement of the disfiguring enophthalmos condition.

### Brief description of the drawings

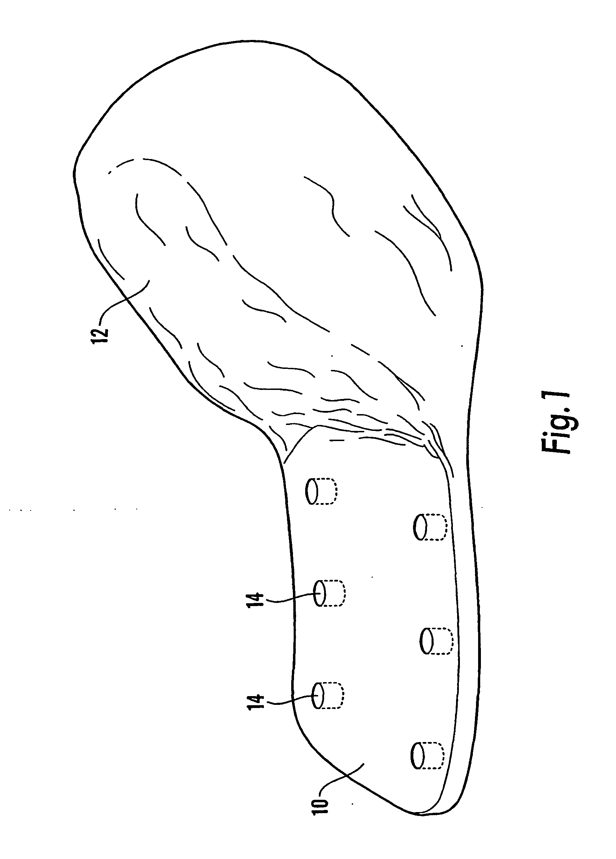

Figure 1 is a perspective view of the orbital implant of the invention;
Figure 2 is a side elevational view of the orbital implant of the invention;
Figure 3 a bottom plan view of the orbital implant of the invention;
Figure 4 is a perspective view of the orbital implant of the invention;
Figure 5 is a sectional view showing the implant in place in an eye socket occupied by an ocular implant and prosthesis.

### Detailed description of the invention

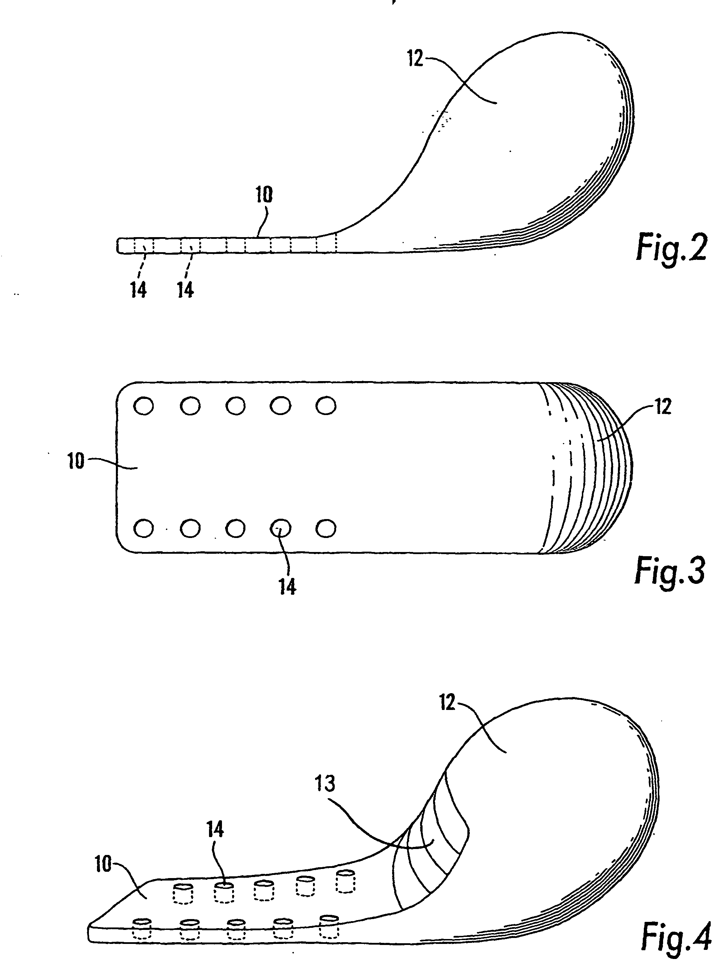

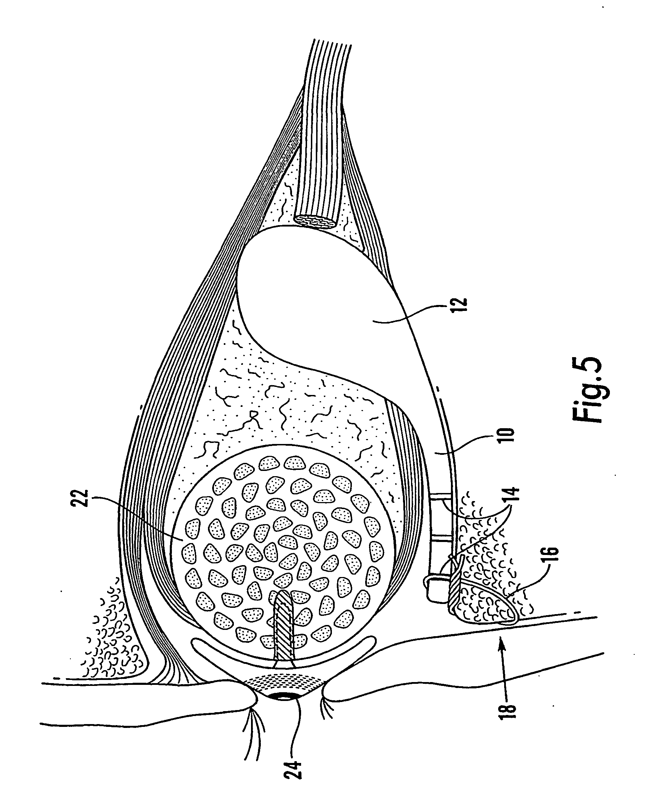

As shown in the figures, the implant according to the invention comprises a plate 10 with a posterior, generally spherical projection 12. Plate 10 further comprises two rows of a plurality of attachment holes 14. As can be appreciated from Fig. 3, the length of plate 10 may be adjusted by cutting away a portion of the anterior end of the plate. Fig. 5 illustrates the implant according to the invention affixed to the orbital floor by a stainless-steel ligature 16. As can be discerned from figure 5, ligature 16 passes through a drill hole 18 in the inferior orbital rim. It is to be understood, however, that the implant could alternatively be attached by screws. The implant is positioned behind an ocular implant 22 to which is connected an eye prosthesis 24. As can be appreciated, spherical projection 12 serves to replace lost soft-tissue volume and press ocular implant 22 forward, thus mitigating the enophthalmos condition. According to the preferred embodiment, projection 12 is generally spherical, however any appropriate, space-filling shape could be employed.

## Claims

1. A supplemental orbital implant for ameliorating enophthalmos, comprising an elongated plate (10) having an anterior and a posterior end, and a space-filling projection (12) extending above the plane defined by the plate, **characterized in that**:
- the plate (10) has dimensions which are adapted to the dimensions of the orbital floor, and
- space-filling projection (12) is an extension of the posterior end of the plate and has dimensions predetermined to approximate the lost soft-tissue volume immediately behind an artificial spherical eye implant (22) that has been surgically implanted following eye enucleation,
whereby, when inserted in the orbital socket, the space-filling projection exerts forward pressure on artificial eye implant (22).

2. An orbital implant according to claim 1, wherein said space-filling projection (12) is generally spherical.

3. An orbital implant according to claim 1, wherein said space-filling projection is bulbous.

4. An orbital implant according to claim 2, wherein plate (10) has a plurality of attachment holes (14) arranged in pairs in at least two rows at the anterior end of the plate.

## Patentansprüche

1. Ergänzendes Augenhöhlen-Implantat zum Verbessern von Enophtalmie, aufweisend eine längliche Platte (10), welche ein vorderes und hinteres Ende aufweist, und einen raumfüllenden Vorsprung (12), der sich über die Ebene erstreckt, welche durch die Platte definiert wird, **dadurch gekennzeichnet, dass**:
- die Platte (10) Abmessungen aufweist, welche angepasst sind auf die Abmessungen des Augenhöhlenbodens (bzw. Orbitalbodens), und
- der raumfüllende Vorsprung (12) eine Erweiterung des hinteren Endes der Platte ist und vorbestimmte Abmessungen aufweist, um sich dem verlorenen Volumen von weichem Gewebe unmittelbar hinter einem künstlichen, sphärischen Augenimplantat (22) anzunähern, das chirurgisch nachfolgend auf eine Augen-Enukleation implantiert worden ist,
wobei der raumausfiillende Vorsprung, wenn er in die Augenhöhle eingeführt wird, einen Druck nach vorne auf das künstliche Augenimplantat (22) ausübt.

2. Augenhöhlen-Implantat nach Anspruch 1, wobei der raumfüllende Vorsprung (12) im Allgemeinen kugelförmig ist.

3. Augenhöhlen-Implantat nach Anspruch 1, wobei der raumfüllende Vorsprung knollenförmig ist.

4. Augenhöhlen-Implantat nach Anspruch 2, wobei die Platte (10) eine Mehrzahl von Befestigungslöchern (14) aufweist, die in Paaren von mindestens zwei Reihen an dem vorderen Ende der Platte angeordnet sind.

## Revendications

1. Implant orbitaire additionnel pour améliorer l'énophtalmie, comprenant une plaque allongée (10) ayant une extrémité antérieure et une extrémité postérieure, et une projection remplissant l'espace (12) s'étendant au-dessus du plan défini par la plaque, **caractérisé en ce que** :
- la plaque (10) a des dimensions qui sont adaptées aux dimensions du plancher orbital, et
- une projection remplissant l'espace (12) est une extension de l'extrémité postérieure de la plaque et a des dimensions prédéterminées pour être proches du volume de tissu mou perdu immédiatement derrière un implant oculaire sphérique artificiel (22) qui a été implanté par chirurgie après énucléation de l'oeil,
sachant que, lors de l'implantation dans l'orbite, la projection remplissant l'espace exerce une pression vers l'avant sur l'implant oculaire artificiel (22).

2. Implant orbitaire selon la revendication 1, dans lequel ladite projection remplissant l'espace (12) est généralement sphérique.

3. Implant orbitaire selon la revendication 1 dans lequel ladite projection remplissant l'espace est bulbeuse.

4. Implant orbitaire selon la revendication 2, dans lequel la plaque (10) a une pluralité d'orifices de fixation (14) arrangés par paires en au moins deux rangées à l'extrémité antérieure de la plaque.
